# EUROPEAN PATENT APPLICATION

(11) **EP 4 431 095 A1**
(43) Date of publication of application: **18.09.2024**
(21) Application number: 22889775.7
(22) Date of filing: 19.10.2022
(51) Int. Cl.: A61K 31/4172, A23L 33/10, A23L 33/14, A61K 36/06, A61K 47/40, A61P 27/16

(54) **ERGOTHIONEINE-CONTAINING COMPOSITION FOR SUPPRESSING OR PREVENTING INNER EAR HEARING LOSS**

(30) Priority: 08.11.2021 JP 2021181678
(71) Applicant: MITSUBISHI GAS CHEMICAL COMPANY, INC., Chiyoda-ku Tokyo 100-8324 (JP)
(72) Inventor: TOIDA, Takumi, Niigata-shi, Niigata 950-3112 (JP); GAYAMA, Shinyo, Niigata-shi, Niigata 950-3112 (JP)
(74) Representative: Müller-Boré & Partner Patentanwälte PartG mbB
(86) International application number: PCT/JP2022/038850
(87) International publication number: WO 2023/079954

(57) **Abstract**

The present invention provides a composition for suppressing or preventing inner ear hearing loss, containing ergothioneine or a salt thereof.

## Description

### Technical Field

The present invention relates to novel use of ergothioneine, particularly use for suppressing or preventing inner ear hearing loss.

### Background Art

Ergothioneine, a type of amino acids, is known to have a high antioxidant effect, but is a substance that cannot be synthesized in the human body, and therefore, ergothioneine is expected to be developed into cosmetics, food, pharmaceuticals, feed and the like in which ergothioneine is blended. The function performed by ergothioneine in the human body has, however, not been clarified yet.

Although it has been reported that the antioxidant effect of ergothioneine is superior to those of other antioxidants such as glutathione, not all antioxidants are useful for treating or preventing diseases caused by oxidation occurring in the body, and it is necessary to individually examine the antioxidants for finding the efficacies, the effects or pharmaceutical applications.

### Citation List

### Patent Literature

Patent Literature 1: Japanese Patent No. 5421366

### Summary of Invention

### Technical Problem

Most cases of hearing loss have a disorder in any one of the outer ear, the middle ear, and the inner ear which make up the ear, and the hearing loss is broadly classified, depending on the part, into two types of hearing loss: conductive hearing loss (the outer ear, and the middle ear), and sensorineural hearing loss (the inner ear). These hearing losses are further subclassified depending on the cause, and age-related hearing loss is known as one type of sensorineural hearing loss.

Age-related hearing loss is literally hearing loss progressing with age, and there is no curative treatment method therefor.

Japanese Patent No. 5421366 discloses a sterile pharmaceutical composition for use for treatment of an ear disease through intratympanic injection, in which the pharmaceutical composition comprises a thermoreversible aqueous gel containing a copolymer of polyoxypropylene and polyoxyethylene, which is acceptable for the ear, and an ear structure modifier that can be slow-released to the ear at least for 3 days, and the ear structure modifier is a bone remodeling modulator (Claim 1 and the like).

This publication enumerates various diseases as the target disease of the composition, and hearing loss or age-related hearing loss is included therein, but actually, an experiment for confirming that the pharmaceutical composition is effective for prevention and the like of inner ear hearing loss such as age-related hearing loss is not conducted.

There are various causes of hearing loss, and it is unknown whether or not a substance effective for preventing one hearing loss is also effective for another hearing loss.

### Solution to Problem

Surprisingly, the present inventors have found that ergothioneine is effective for prevention and the like of inner ear hearing loss such as age-related hearing loss, and have completed the present invention.

Specifically, the present application encompasses the following inventions:
[1] A composition for suppressing or preventing inner ear hearing loss, comprising ergothioneine.
[2] The composition according to [1], wherein the ergothioneine is administered in an amount ranging from 0.01 mg/kg of body weight or more to less than 100 mg/kg of body weight per day.
[3] The composition according to [2], wherein the ergothioneine is administered once or a plurality of times a day.
[4] The composition according to [2] or [3], wherein an administration period is at least 1 week or more.
[5] The composition according to any one of [2] to [4], wherein the administration includes oral administration.
[6] The composition according to any one of [1] to [5], wherein the inner ear hearing loss is age-related hearing loss.
[7] The composition according to any one of [1] to [6], wherein the inner ear hearing loss is not drug-induced hearing loss.
[8] The composition according to any one of [1] to [7], wherein a subject to whom the ergothioneine is to be administered has been diagnosed with age-related hearing loss through auditory brainstem response (ABR).
[9] The composition according to any one of [1] to [8], wherein the ergothioneine is derived from a yeast.
[10] The composition according to [9], wherein the yeast is an imperfect yeast belonging to genus *Rhodotorula.*
[11] The composition according to [8] or [9], further comprising a yeast extract.
[12] The composition according to [11], wherein the ergothioneine is contained in the yeast extract.
[13] The composition according to [12], wherein the ergothioneine is contained in the yeast extract in an amount of 0.1 to 20% in terms of a concentration in a solid content.
[14] The composition according to any one of [11] to [13], wherein the yeast extract is a powder.
[15] The composition according to any one of [1] to [14], further comprising cyclodextrin.
[16] The composition according to any one of [1] to [15], wherein the composition is in a form of a pharmaceutical, a food, or a supplement.
[17] The composition according to [16], wherein the ergothioneine is administered in an amount ranging from 0.1 mg or more to less than 1000 mg per dose per day.
[18] A method for suppressing or preventing inner ear hearing loss in a subject, comprising administering ergothioneine to the subject.
[19] The method according to [18], wherein the ergothioneine is administered once or a plurality of times a day.
[20] The method according to [18] or [19], wherein an administration period is at least 1 week or more.
[21] The method according to any one of [18] to [20], wherein the administration includes oral administration.

### Advantageous Effects of Invention

Since the safety of ergothioneine in a human body has been confirmed, inner ear hearing loss can be expected to be prevented by the present invention by blending ergothioneine in a supplement and the like.

### Description of Embodiments

A preferred embodiment of the present invention (hereinafter referred to as the "present embodiment") will be described below, and it should be noted that the scope of the present invention is not understood to be limited to the following embodiment.

### (Composition)

A first embodiment provides a composition for suppressing or preventing inner ear hearing loss comprising ergothioneine or a salt thereof.

The causes of inner ear hearing loss include congenital causes and acquired causes. Age-related hearing loss, which is known also as presbycusis, is an acquired inner ear hearing loss, and categorized into sensorineural hearing loss. Inner ear hearing loss is preferably age-related hearing loss. Ergothioneine has an effect of suppressing hearing loss in a low frequency region of an audible range (for example, 1 to 40 kHz for a mouse, and 2 to 20 kHz for a human), preferably in a 0% to 90% frequency band from a low frequency region side in the audible range (for example, 1 to 36.1 kHz for a mouse, and 2 to 18.2 kHz for a human), and more preferably in a 0% to 70% frequency band therefrom (for example, 1 to 28.3 kHz for a mouse, and 2 to 14.6 kHz for a human). As used herein, the phrase "0% to 90% frequency band from a low frequency region side in the audible range" refers to a value obtained by multiplying, by a corresponding percentage, [lower limit frequency] + ([upper limit frequency] - [lower limit frequency]), and for example, assuming that the lower limit frequency is 1 kHz and the upper limit frequency is 40 kHz, a 50% frequency is 20.5 {(1 + (40 - 1) x 0.5)}.

As the other causes of acquired inner ear hearing loss, those caused by genetic diseases (such as mutations in the connexin 26 gene, Waardenburg syndrome, Usher syndrome, and Pendred syndrome), exposure to noise, ototoxic drugs (such as aspirin, aminoglycoside drugs, vancomycin, cisplatin, furosemide, ethacrynic acid, and quinine), infectious diseases (such as meningitis, and purulent labyrinthitis), autoimmune diseases (such as rheumatoid arthritis, and systemic lupus erythematosus), Meniere syndrome, barotrauma (accompanied by perilymphatic fistula), head injury (accompanied by skull base fracture, or concussion of cochlea), and auditory neuropathy (auditory nerve disease) are known. These acquired inner ear hearing losses, particularly hearing losses induced by drugs, are clearly distinguished from age-related hearing loss.

Examples of congenital inner ear hearing loss include those caused by hereditary or idiopathic (cryptogenic) anomaly, congenital infections diseases (such as rubella, cytomegalovirus infection, toxoplasmosis, and syphilis), rhesus incompatibility, anoxia, maternal uptake of ototoxic drugs (such as those for tuberculosis, and severe infection), and drug-induced anomaly (such as those induced by thalidomide).

Ergothioneine used as the active ingredient is a type of sulfur-containing amino acids. When the composition containing an effective amount of ergothioneine is taken, inner ear hearing loss is suppressed, prevented, or treated. Ergothioneine is preferably an L-isomer.

Ergothioneine may be in the form of a salt. The type of the salt is not particularly limited as long as it is pharmaceutically acceptable, and examples include salts with hydrochloric acid, phosphoric acid, sulfuric acid, nitric acid, boric acid and the like (inorganic acid salts), and salts with formic acid, acetic acid, lactic acid, fumaric acid, maleic acid, tartaric acid, citric acid and the like (organic acid salts). The term "ergothioneine" as used herein can encompass the pharmaceutically acceptable salts thereof unless otherwise stated.

Ergothioneine may be a commercially available product, or may be synthesized. Examples of a method for producing ergothioneine include a chemical synthesis method, a method in which a fungus producing ergothioneine (including mushrooms such as shaggy mane, oyster mushroom, golden oyster mushroom, enoki mushroom, king trumpet mushroom, and shiitake mushroom) is cultured for extracting and purifying ergothioneine therefrom, a fermentation method, and a method in which ergothioneine is extracted from animal blood or the like containing it.

An example of the fungus producing ergothioneine includes a yeast. The term "yeast" as used herein is not particularly limited as long as it can produce ergothioneine under culture conditions. The yeast herein is preferably a budding yeast, and more preferably an imperfect yeast.

Examples of the imperfect yeast include yeasts belonging to the genus *Rhodotorula,* the genus *Cryptococcus* and the like, among which those belonging to the genus *Rhodotorula* are preferred. The yeasts belonging to the genus *Rhodotorula* encompass *Rhodotorula mucilaginosa, Rhodotorula glutinis* and the like.

Ergothioneine, when derived from a fungus such as a yeast, may be blended in the composition in a state where it is contained in the fungus, or may be blended in the composition in the form of an extract after being extracted from the fungus. Ergothioneine is contained in the fungus or the extract in an optional concentration. For example, the extract may contain ergothioneine in an amount of 0.1 to 20% in terms of a concentration in a solid content. Ergothioneine can be appropriately purified in accordance with a desired purity. Any known method can be employed as the purification method, and for example, column chromatography using a normal phase column, a reverse phase column, an ion exchange column, or the like, crystallization, membrane treatment, activated carbon treatment, and the like can be performed in an appropriate combination.

The composition may be in the form of the fungus cells, or may be present in a form of a pharmaceutical, a food, or a supplement in combination with another components. Examples of the food include food for specified health uses, food with functional claims, and food with nutrient function claims. The composition may contain, as long as the effect of ergothioneine as the active ingredient is not impaired, an additional component for suppressing or preventing inner ear hearing loss, and a usually used carrier, such as an excipient, a disintegrating agent, a lubricant, a buffer, a binder, an emulsifier, a suspending agent, a stabilizer, a preservative, an antiseptic, or saline. An example of the stabilizer includes cyclodextrin. As the cyclodextrin, α-cyclodextrin, β-cyclodextrin, γ-cyclodextrin, or a combination thereof can be used, and from the viewpoint of increasing the effect of suppressing or preventing inner ear hearing loss, γ-cyclodextrin is preferred. When ergothioneine is derived from a fungus, the composition may contain the fungus cells or extract thereof, or can contain purified ergothioneine. The extract of the fungus may be in the form of a powder.

The subject to whom the composition is to be administered is not limited to a human, but may be a non-human animal, such as a mouse. The composition is administered to a subject who is expected to develop inner ear hearing loss in the future, or a subject who has already developed inner ear hearing loss, for example, a subject having been diagnosed as suffering from inner ear hearing loss. A method for diagnosing inner ear hearing loss is not particularly limited, and an example of a method for diagnosing age-related hearing loss includes auditory brainstem response (ABR).

The amount of ergothioneine in the composition is appropriately changed in accordance with the symptom, the age, and the sex of a human to whom the composition is to be administered, the dosing frequency, and the like, and can be appropriately adjusted within a range of 0.01 mg/kg of body weight or more to less than 100 mg/kg of body weight per day. For a human, ergothioneine is preferably administered in an amount of about 0.1 mg to 1000 mg per day, ergothioneine is more preferably administered in an amount of about 0.2 mg to 800 mg, and ergothioneine is particularly preferably administered in an amount of about 0.5 mg to 500 mg.

An administration period and the dosing frequency are varied depending on the symptom of the subject and a dose, and it is preferable to administer it once a day at least for 1 week or more, preferably for 10 weeks or more, for example, for 13 weeks or more. For hearing loss in a high frequency band of about 32 kHz, the effect of suppressing hearing loss can be increased through long-term treatment, for example, the treatment for 13 weeks or more.

Examples of a dosage form of the composition include a tablet, a powder, a fine granule, a granule, a capsule, a syrup, an injection, an external preparation, and a suppository.

As the excipient, lactose, starch, sorbitol, D-mannitol, or the like can be used. As the disintegrating agent, starch, carboxymethylcellulose, calcium carbonate, or the like can be used. As the buffer, phosphate, citrate, acetate, or the like can be used. As the emulsifier, gum arabic, sodium alginate, tragacanth, or the like can be used. As the binder, pullulan, gum arabic, gelatin, starch, or the like can be used. As the lubricant, magnesium stearate, methylcellulose, or magnesium silicate can be used. As the suspending agent, glycerin monostearate, aluminum monostearate, methylcellulose, carboxymethylcellulose, hydroxymethylcellulose, sodium lauryl sulfate, or the like can be used. As the stabilizer, propylene glycol, diethyline sulfite, ascorbic acid, or the like can be used. As the preservative, phenol, benzalkonium chloride, benzyl alcohol, chlorobutanol, methylparaben, or the like can be used. As the antiseptic, benzalkonium chloride, parahydroxybenzoate, chlorobutanol, or the like can be used.

The administration method of the composition is not particularly limited as long as inner ear hearing loss can be suppressed or prevented, and examples include oral administration, and parenteral administration such as intravenous, intraperitoneal, subcutaneous, or transdermal administration.

### (Method)

A second embodiment provides a method for suppressing or preventing inner ear hearing loss in a subject, including administering ergothioneine or a salt thereof to the subject.

The subject to whom ergothioneine is to be administered is one in need of suppression or prevention of inner ear hearing loss. The subject is not limited to a human, but may be a non-human animal, such as a mouse.

Ergothioneine can be administered in combination with other components known to have an effect of suppressing or preventing inner ear hearing loss, and such components may be administered at the same time, or at different timing.

The dosage form, the dose, the administration route and the like of ergothioneine are the same as those described above.

Hereinafter, the present invention will be more specifically described by way of examples, and it is noted that the present invention is not limited to these examples.

### Examples

### Example 1:

### 1. Materials and Methods

### 1.1. Ergothioneine and Medium

### 1.1.1. Production of Yeast Extract Powder Containing Ergothioneine

From a culture fluid obtained by culturing *Rhodotorula mucilaginosa* yeast in a liquid medium, a fungus cells were collected by centrifugation (12,000 x g, 90 min). The thus obtained fungus cells were washed with water, and subjected to centrifugation (12,000 x g, 90 min) repeatedly twice. Water was added to the resultant fungus cells, and the resultant was treated at 95°C for 10 minutes to extract ergothioneine from the fungus cells. Thereafter, centrifugation (12,000 x g, 90 min) was performed to obtain an extract, which was subjected to UF membrane (molecular weight cut off: 6 kDa, 25°C) treatment to obtain a crude purified liquid. The crude purified liquid was concentrated with an evaporator, followed by heat sterilization treatment at 80°C for 30 minutes. The crude purified liquid after the sterilization treatment was spray dried to obtain a yeast extract powder.

In this example, the ergothioneine content was measured by HPLC using Asahipak NH2P-50 column. Ergothioneine was eluted using a concentration gradient liquid of an eluate A (0.1 vol% triethylamine, 50 mM sodium phosphate buffer: pH 7.3) and an eluate B (100 mM NaCl). The measurement was performed with an absorbance at a wavelength of 254 nm, and ergothioneine elution was found at about 5.7 min. An aqueous solution containing the yeast extract in a concentration of 10 µg/ml was membrane filtered with a 0.2 µm filter, the resultant filtrate was subjected to HPLC measurement, and thus, it was confirmed that ergothioneine was contained in an amount of 75 mg/g.

### 1.1.2. Medium

Water for injection (manufactured by Otsuka Pharmaceutical)

### 1.2. Anesthetic

A mixture of necessary amounts of the following reagents was used.
- Ketamine hydrochloride (KETALAR(R) for intravenous injection 500 mg, Daiichi Sankyo Propharma Co., Ltd.)
- Xylazine hydrochloride (Selactar(R) 2% for injection, Bayer Yakuhin, Ltd.)

### 1.3. Administration Sample

### 1.3.1. Preparation Method

### 1.3.1.1. Preparation of Yeast Extract Powder Containing Ergothioneine

After weighing 90 mg of the yeast extract powder, the powder was dissolved in the water for injection to prepare a solution having a concentration of the yeast extract powder of 30 mg/mL. Such a solution was prepared at the time of use.

### 1.4.1.2. Mixed Anesthetic

KETALAR(R) for intramuscular injection 500 mg and Selactar(R) 2% for injection were taken in a blending ratio of 1.5 mL and 0.5 mL, respectively, at the time of use and mixed and used.

### 2. Test System

### 2.1. Animal Species, and Strain

The following mouse (SPF) strain, that is, animal species generally used as age-related hearing loss model, and having established strain maintenance, was used.
C57BL/6J (Charles River Laboratories Japan, Inc.)
2.2. Sex, Age, and Number of Obtained Animals
Male, 10 weeks old, 20 mice

### 2.2 Preliminary Rearing

After obtaining, the animals were preliminarily reared for 14 days. During this period, the body weight was measured 3 times, the ABR test was performed once, the general status was observed once a day, and animals having no abnormality in body weight change, the ABR test, and the general status were used for grouping.

### 2.3. Grouping

3 cases of animals found to have high thresholds of the sound pressure at 24 and 32 kHz in the ABR test performed during the preliminary rearing period were excluded (not excluded if 4 or more cases were found), and a computer program (IBUKI, Nihon Bioresearch Inc.) was used for stratifying the body weights of the animals, and then, the animals were divided into groups using random sampling method so that the variance in body weight distribution in each group was approximately equal.

### 2.4. Environmental Conditions and Rearing Management

The animals were reared in an animal room maintained at a controlled temperature: 18 to 28°C, a controlled humidity: 30 to 80%RH, 12-hour each light and dark cycle (light: 6 o'clock to 18 o'clock), and air exchange rate: 12 times/hour (fresh air through a filter).

### 3. Administration

### 3.1. Administration Route, Administration Method, Administration Time, Amount of Administered Liquid, Administration Period, and Administration Frequency

### Administration route: oral

Administration method: A sample was orally administered using a polypropylene disposable syringe (Terumo Corporation) equipped with a disposable feeding needle for mice (Fuchigami Kikai). At the time of the administration, the sample was stirred with a magnetic stirrer, and aspirated into the syringe, and then, a portion of the sample attaching to the feeding needle was wiped off.

Administration time: The administration was performed from 8:30 to 11:00.

Amount of administered liquid: The amount was calculated as 10 mL/kg based on the body weight measured on a date closest to the administration date.
Administration period: 13 weeks (91 days)
Administration frequency: once a day

### 4. Groups

Test groups were the following two groups. The number of animals of each group was 8.

**[Table 1]**

| Group number | Group name | Dose (mg/kg) | Number of animals |
|---|---|---|---|
| 1 | Control | 0^{a)} | 8 |
| 2 | Yeast extract powder | 300 | 8 |

| | | | |
|---|---|---|---|
| a) The medium was administered in an amount of 10 mL/kg. | | | |

Individual animals having final numbers of animal numbers of 1 to 4 in each group were allocated to a first course, and those having final numbers of 5 to 8 were allocated to a second course.

### 5. Observation and Test

### 5.1. Observation of General Status

The observation was performed once every day in the morning (basically about 1 hour after the administration).

### 5.2. Body Weight Measurement

The measurement was performed on day 1 of administration (with the first date of administration counted as day 1), and thereafter, once every 2 weeks, and at the time of the observation of the general status on a date of the ABR test.

### 5.3. ABR (auditory brainstem response) Test

The ABR test was performed before the administration (11 weeks old), and on a next day of the final administration date (25 weeks old). The mixed anesthetic [ketamine hydrochloride (75 mg/kg) and xylazine hydrochloride (10 mg/kg)] was subcutaneously administered (amount of administered liquid: 2 mL/kg) to each animal with a polypropylene disposable syringe (Terumo Corporation) equipped with a 27G injection needle (Terumo Corporation). After the anesthesia, a different electrode was subcutaneously attached in the vicinity of the outer ear of the ear to be tested (right ear), an indifferent electrode was subcutaneously attached to a parietal region, and a ground electrode was subcutaneously attached to the back of the neck. ABR potential was induced from the electrodes to a biopotential amplifier (Model: ER-1, Cygnus Technology Inc.) to be recorded in a data record/analysis system (PowerLab, Sampling soft: LabChart ver. 8, AD Instruments) (recording conditions: sampling time: 10 ms, sampling rate: 40 kHz, Bandpass filter: 1 to 3000 Hz, number of acquisitions: 500 times).

Sound stimulation was given using TDT sound system (ZBus for system 3, Tucker-Davis Technologies Inc.) with Coupler type speaker (Model: ES1spc, Bioresearch Inc.) inserted into the right ear canal (range of sound pressure: 10 to 90 dB, type of sound: tone burst at 24 and 32 kHz). First, sound stimulation with 90 dB was given, and the ABR was recorded. Thereafter, the sound pressure was appropriately changed, in increments of 5 dB, to confirm a maximum sound pressure at which the ABR waveform disappeared, and a minimum sound pressure at which the ABR waveform was detected. The minimum sound pressure at which the ABR waveform was detected was defined as the sound pressure threshold (ABR threshold: dB SPL). When the ABR waveform was not detected with the sound stimulation of 90 dB, the sound pressure threshold was regarded as 95 dB SPL. After the measurement, the ABR waveform data was stored in an electronic medium (CD-R) .

### 7. Results

As shown in Table 2 (results of the ABR threshold at 24 kHz) and Table 3 (results of the ABR threshold at 32 kHz) below, in a mouse to which the extract powder containing ergothioneine was administered in an amount of 300 mg per kg of body weight per day (ergothioneine content: 7.5%) for 13 weeks, change of an average ABR threshold was reduced as compared with that of a control group.

**[Table 2]**

| Group | Control | Yeast extract powder |
|---|---|---|
| Dose (mg/kg) | 0 | 300 |
| Number of animals | 8 | 8 |
| Average ABR threshold (dB SPL)±S.E.) | | |
| Before administration | 36±2 | 35±2 |
| 13 Weeks from start of administration | 54±6 | 44±5 |

**[Table 3]**

| Group | Control | Yeast extract powder |
|---|---|---|
| Dose (mg/kg) | 0 | 300 |
| Number of animals | 8 | 8 |
| Average ABR threshold (dB SPL)±S.E.) | | |
| Before administration | 54±3 | 51±4 |
| 13 Weeks from start of administration | 76±3 | 69±2 |

### Example 2:

A yeast extract powder was prepared in the same manner as in Example 1 except that γ-cyclodextrin was added thereto so that the solid content becomes 20 parts by weight when spray dried. The thus obtained yeast extract powder was subjected to HPLC measurement in the same manner as in Example 1 to confirm that the powder contains 58 mg/g of ergothioneine. Procedures thereafter were the same as those of Example 1, and although the yeast extract powder containing ergothioneine was prepared as a liquid of a concentration of 38.8 mg/mL different from 30 mg/mL of Example 1, the amount of ergothioneine to be administered to each animal was the same as that of Example 1.

In a test system using animal models, 10 weeks old males were used as in Example 1, but the number of animals was increased to 28. Besides, the preliminary rearing period was 15 days. Test groups were the following 2 groups. The number of animals of each group was 12.

**[Table 4]**

| Group number | Group name | Dose (mg/kg) | Number of animals |
|---|---|---|---|
| 3 | Control | 0^{a)} | 12 |
| 4 | Yeast extract powder | 388 | 12 |

| | | | |
|---|---|---|---|
| a) The medium was administered in an amount of 10 mL/kg. | | | |

After the administration, the animals were subjected to observation and ABR (auditory brainstem response) test in the same manner as in Example 1. These were performed before the administration (11 weeks old), in the 8th week of the administration (20 weeks old), and on a next day of the final administration date (25 weeks old), which was only difference from Example 1. Averages and standard deviations of the sound pressure threshold and the body weight at each time point of each group were calculated, and the thus obtained results were statistically analyzed. As a significance test, Wilcoxon signed-rank test was first performed on the sound pressure thresholds at 24 and 32 kHz between 11 weeks old (pre) animals and 25 weeks old (post) animals of the control group, and it was thus confirmed that hearing loss had been caused in an age-related manner. Next, Wilcoxon signed-rank test was performed at each time point between the control group and the test substance group. A significance level was set to 5%, and results are shown dividedly as less than 5% (p < 0.05) and less than 1% (p < 0.01). In the significance test, a commercially available statistical program (SAS System, SAS Institute Japan) was used.

As a result, as shown in the following Table 5 (results of the ABR threshold at 24 kHz) and Table 6 (results of the ABR threshold at 32 kHz), in a mouse to which the extract powder containing ergothioneine was administered in an amount of 388 mg per kg of body weight per day (ergothioneine content: 5.8%) for 13 weeks, change of an average ABR threshold was reduced as compared with that of the control group.

**[Table 5]**

| Group | Control | Yeast extract powder |
|---|---|---|
| Dose (mg/kg) | 0 | 388 |
| Number of animals | 12 | 12 |
| Before administration | 39±2 | 38±2 |
| 8 Weeks from start of administration | 53±3 | 40±4 ## |
| 13 Weeks from start of administration | 61±5 ** | 47±5 # |

| | | |
|---|---|---|
| Each value indicates an average ABR threshold (dB SPL) ± S.E.) in the same manner as in Example 1. The same shall apply hereinafter. **: p < 0.01, there is a significant difference as compared with that before administration. #: p < 0.05, ##: p < 0.01, there is a significant difference as compared with the control. | | |

**[Table 6]**

| Group | Control | Yeast extract powder |
|---|---|---|
| Dose (mg/kg) | 0 | 388 |
| Number of animals | 12 | 12 |
| Before administration | 55±3 | 49±3 |
| 8 Weeks from start of administration | 70±2 | 56±5 # |
| 13 Weeks from start of administration | 72±2 ** | 63±4 |

| | | |
|---|---|---|
| **: p < 0.01, there is a significant difference as compared with that before administration. #: p < 0.05, there is a significant difference as compared with the control. | | |

As shown in Tables 5 and 6, the hearing loss suppression effect of ergothioneine was confirmed at either frequency, but was particularly remarkable at 24 kHz. Since the symptom of hearing loss usually develops from a high sound area (Exp Anim. 2015; 64(3): 241-251), the suppression effect is difficult to obtain at a high frequency (32 kHz) including variations, but the hearing loss suppression effect is increased for the high frequency by extending the administration period.

It is understood, from the above-described results, that progression of hearing loss was delayed by ergothioneine, and as a result, at the measurement timing, the ABR waveform was not detected at a high frequency band in the ergothioneine administration group, but the ABR waveform was detected at a low frequency band. In other words, it is presumed that when ergothioneine is not administered, an audible frequency is reduced in a short time, but when ergothioneine is administered, the reduction rate is delayed.

## Claims

1. A composition for suppressing or preventing inner ear hearing loss, comprising ergothioneine.

2. The composition according to claim 1, wherein the ergothioneine is administered in an amount ranging from 0.01 mg/kg of body weight or more to less than 100 mg/kg of body weight per day.

3. The composition according to claim 2, wherein the ergothioneine is administered once or a plurality of times a day.

4. The composition according to claim 2 or 3, wherein an administration period is at least 1 week or more.

5. The composition according to any one of claims 2 to 4, wherein the administration includes oral administration.

6. The composition according to any one of claims 1 to 5, wherein the inner ear hearing loss is age-related hearing loss.

7. The composition according to any one of claims 1 to 6, wherein the inner ear hearing loss is not drug-induced hearing loss.

8. The composition according to any one of claims 1 to 7, wherein a subject to whom the ergothioneine is to be administered has been diagnosed with age-related hearing loss through auditory brainstem response (ABR).

9. The composition according to any one of claims 1 to 8, wherein the ergothioneine is derived from a yeast.

10. The composition according to claim 9, wherein the yeast is an imperfect yeast belonging to genus *Rhodotorula.*

11. The composition according to claim 8 or 9, further comprising a yeast extract.

12. The composition according to claim 11, wherein the ergothioneine is contained in the yeast extract.

13. The composition according to claim 12, wherein the ergothioneine is contained in the yeast extract in an amount of 0.1 to 20% in terms of a concentration in a solid content.

14. The composition according to any one of claims 11 to 13, wherein the yeast extract is a powder.

15. The composition according to any one of claims 1 to 14, further comprising cyclodextrin.

16. The composition according to any one of claims 1 to 15, wherein the composition is in a form of a pharmaceutical, a food, or a supplement.

17. The composition according to claim 16, wherein the ergothioneine is administered in an amount ranging from 0.1 mg or more to less than 1000 mg per dose per day.

18. A method for suppressing or preventing inner ear hearing loss in a subject, comprising administering ergothioneine to the subject.

19. The method according to claim 18, wherein the ergothioneine is administered once or a plurality of times a day.

20. The method according to claim 18 or 19, wherein an administration period is at least 1 week or more.

21. The method according to any one of claims 18 to 20, wherein the administration includes oral administration.
